(19) **Europäisches Patentamt / European Patent Office / Office européen des brevets**

(11) **EP 4 458 410 A1**

(12) # EUROPEAN PATENT APPLICATION

(43) Date of publication:
**06.11.2024 Bulletin 2024/45**

(21) Application number: **23191565.3**

(22) Date of filing: **15.08.2023**

(51) International Patent Classification (IPC):
**A61N 7/00** *(2006.01)*   **A61N 7/02** *(2006.01)*

(52) Cooperative Patent Classification (CPC):
**A61N 7/02; A61N 7/00;** A61N 2007/0039;
A61N 2007/0073

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.07.2023 KR 20230090886**

(71) Applicant: IMGT Co, Ltd.
**Seongnam-si, Gyeonggi-do 13605 (KR)**

(72) Inventors:
• **SON, Keon Ho**
  **13462 Gyeonggi-do (KR)**
• **KIM, Dae Seung**
  **07524 Seoul (KR)**
• **KOO, Ja Woon**
  **10063 Gyeonggi-do (KR)**

(74) Representative: **Sander, Rolf**
  **IPNY AB**
  **Birger Jarlsgaten 99A**
  **11356 Stockholm (SE)**

(54) **FOCUSED ULTRASOUND PROCESSING APPARATUS**

(57) Provided are a focused ultrasound processing apparatus and a method thereof. The focused ultrasound processing apparatus in one general aspect includes a focused ultrasound transducer configured to output a focused ultrasound signal to tissue, a cavitation sensor configured to detect a cavitation signal resulting from a cavitation phenomenon occurring in the tissue due to the focused ultrasound signal, and a processor configured to analyze the cavitation signal detected by the cavitation sensor and adjust at least one of output time or frequency of the focused ultrasound signal based on an analysis result.

FIG. 1

## Description

## BACKGROUND

### 1. Field

The present invention relates to diagnosis and treatment technology using ultrasound, and more particularly, to image scanning and treatment technology using focused ultrasound (FUS).

### 2. Description of Related Art

Ultrasound signals may be used in the treatment of biological tissues, such as cancer, tumors, lesions, and the like. Treatment with ultrasound is a method of treating a lesion by outputting ultrasound signals to the lesion of the human body. Ultrasound treatment may cause less trauma of a patient, compared to general surgical treatment or chemotherapy, and realize non-invasive treatment. Examples of the application of ultrasound treatment include liver cancer, bone sarcoma, breast cancer, pancreatic cancer, kidney cancer, soft tissue tumors, pelvic tumors, and the like.

Ultrasound signals may be used in the treatment of tissues, such as cancers, tumors, lesions, and the like. Treatment using FUS signals have primarily focused on developing thermal ablation techniques, which thermally ablates tissues using thermal effects. However, thermal ablation can lead to thermal damage and pain in surrounding tissues.

## SUMMARY

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

The following description relates to a focused ultrasound processing apparatus and a method thereof that enable safer and more effective tissue ablation with focused ultrasound beyond the limits of thermal effects by exploiting mechanical effects.

Furthermore, the following description relates to a focused ultrasound processing apparatus and a method thereof that enable tissue ablation without inducing a shock scattering effect when applying mechanical effects to focused ultrasound tissue ablation.

In one general aspect, there is provided a focused ultrasound processing apparatus comprising a focused ultrasound transducer configured to output a focused ultrasound signal to tissue, a cavitation sensor configured to detect a cavitation signal resulting from a cavitation phenomenon occurring in the tissue due to the focused ultrasound signal, and a processor configured to analyze the cavitation signal detected by the cavitation sensor and adjust at least one of output time or frequency of the focused ultrasound signal based on an analysis result.

The processor may compare a cavitation detection value obtained through the cavitation sensor with a predetermined cavitation threshold value or a cavitation limit value, and based on the comparison result, adjust at least one of the output time or frequency of the focused ultrasound signal in order to decrease the cavitation value.

The processor may compare the cavitation detection value with a boiling cavitation limit value, and adjust at least one of the output time or frequency of the focused ultrasound signal in order to decrease the cavitation detection value when the cavitation detection value is greater than or equal to the boiling cavitation limit value.

The processor may compare the cavitation detection value obtained through the cavitation sensor with at least one of a boiling cavitation threshold value, a non-thermal cavitation limit value, or a non-thermal cavitation threshold value, and adjust at least one of the output time or frequency of the focused ultrasound signal based on the comparison result.

The processor may control output of the focused ultrasound signal by repeating multiple cycles, each cycle may include a first frequency interval consisting of at least one first pulse and a second frequency interval consisting of at least one second pulse, and the first frequency interval may be a thermal period for thermal effects, while the second frequency interval may be a boiling period for mechanical effects.

The first frequency interval may include fh frequency and Ph frequency for thermal effects, and the second frequency interval may include fm frequency and Pm frequency for mechanical effects.

The processor may check a cavitation detection value within a first frequency interval of a first cycle, and may perform control to reduce the number of first pulses in the first frequency interval of a second cycle following the first cycle and increase the number of second pulses of a second frequency interval of the second cycle when the cavitation detection value becomes equal to or exceeds the boiling cavitation limit value.

The processor may check a cavitation detection value within a first frequency interval of a first cycle, and may perform control to reduce the total output time of a second cycle when the cavitation detection value becomes equal to or exceeds a boiling cavitation threshold value, to increase the number of first pulses in a first frequency interval of the second cycle and reduce the number of second pulses in a second frequency interval of the second cycle when the cavitation detection value falls between the boiling cavitation threshold value and a non-thermal cavitation limit value, to maintain the number of first pulses in the first frequency interval of the second cycle and the number of second pulses in the second frequency interval of the second cycle when the cavitation detection value falls between the non-thermal cavitation limit value and a non-thermal cavitation threshold value, and to reduce the number of first pulses in the first frequency interval of the second cycle and increase the number of second pulses in the second frequency interval of the second cycle when the cavitation detection value is less than or equal to the non-thermal cavitation threshold value.

The processor may check each of a cavitation detection value in a first frequency interval of a first cycle and a cavitation detection value in a second frequency interval of the first cycle when primarily outputting the focused ultrasound signal to a first region, and may perform control to move to a new second region output the focused ultrasound signal when the cavitation detection value in the first frequency interval is greater than or equal to a boiling cavitation threshold value and the cavitation detection value in the second frequency interval is greater than or equal to a non-thermal cavitation threshold value, and in other cases, the processor may perform control to secondarily output the focused ultrasound signal to the first region.

The processor may check each of a cavitation detection value in a first frequency interval of a first cycle and a cavitation detection value in a second frequency interval of the first cycle when primarily outputting the focused ultrasound signal to a first region, and may perform control to move to a new second region and output the focused ultrasound signal when the cavitation detection value in the first frequency interval or the cavitation detection value in the second frequency interval is greater than or equal to a non-thermal cavitation threshold value, and in other cases, the processor may perform control to secondarily output the focused ultrasound signal to the first region.

The focused ultrasound processing apparatus may further include an input unit configured to receive tissue characteristics and treatment information, and the processor may determine a cavitation limit value, a cavitation threshold value, and the output time and frequency of the focused ultrasound signal by reflecting the input tissue characteristics and treatment information.

The processor may determine a first frequency for maximizing thermal effect of the focused ultrasound signal before heat generation and a second frequency for maximizing mechanical effect of the focused ultrasound signal after heat generation.

The processor may control a focused ultrasound signal having the determined first frequency to be output through the focused ultrasound transducer, compare a cavitation detection value with at least one of a boiling cavitation limit value or a non-thermal cavitation limit value, and when the cavitation detection value is greater than or equal to the non-thermal cavitation limit value or the boiling cavitation limit value, perform control to adjust a frequency of the focused ultrasound signal from the first frequency to the second frequency and then output the adjusted focused ultrasound signal having the second frequency through the focused ultrasound transducer.

The focused ultrasound processing apparatus may further include an image transducer configured to output an image ultrasound signal to a tissue and receive an ultrasound echo signal reflected from the tissue, and the processor may generate an ultrasound image signal by signal processing the received ultrasound echo signal, analyze the generated ultrasound image signal, and adjust at least one of the output time or frequency of the focused ultrasound signal based on an analysis result.

The processor may control output of the focused ultrasound signal by repeating multiple cycles, each cycle may include a first frequency interval consisting of at least one first pulse and a second frequency interval consisting of at least one second pulse, the first frequency interval may be a thermal period for thermal effects, while the second frequency interval may be a boiling period for mechanical effects, and the processor may check an image brightness value of a focal area in a first frequency interval of a first cycle through analysis of the image signal and perform control to reduce the number of first pulses in a first frequency interval of a second cycle and increase the number of second pulses of a second frequency interval of the second cycle when the image brightness value of the focal area becomes equal to or exceeds a boiling cavitation image brightness limit value.

The processor may control output of the focused ultrasound signal by repeating multiple cycles, each cycle may include a first frequency interval consisting of at least one first pulse and a second frequency interval consisting of at least one second pulse, the first frequency interval may be a thermal period for thermal effects, while the second frequency interval may be a boiling period for mechanical effects, and the processor may check an image brightness value of a focal area in a first frequency interval of a first cycle through analysis of the image signal and perform control to reduce the total output time of a second cycle when the image brightness value of the focal area becomes equal to or exceeds a boiling cavitation image brightness value, to increase the number of first pulses in a first frequency interval of the second cycle and reduce the number of second pulses in a second frequency interval of the second cycle when the image brightness value of the focal area falls between the boiling cavitation threshold value and a non-thermal cavitation image brightness limit value, to maintain the number of first pulses in the first frequency interval of the second cycle and the number of second pulses in the second frequency interval of the second cycle when the image brightness value of the focal area falls between the non-thermal cavitation image brightness limit value and a non-thermal cavitation image brightness threshold value, and to reduce the number of first pulses in the first frequency interval of the second cycle and increase the number of second pulses in the second frequency interval of the second cycle when the image brightness value of the focal area is less than or equal to the non-thermal cavitation image brightness threshold value.

In another general aspect, there is provided a focused ultrasound processing method using a focused ultrasound processing apparatus, the method including determining a first output condition including at least one of output time or frequency of a focused ultrasound signal, outputting a focused ultrasound signal through a focused ultrasound transducer based on the determined first output condition, detecting, by a cavitation sensor, a cavitation signal resulting from a cavitation phenomenon occurring in a tissue due to the output focused ultrasound signal, analyzing a cavitation detection value obtained through the cavitation sensor and adjusting an output condition including at least one of output time or frequency of a focused ultrasound signal to a second output condition based on an analysis result, and outputting the focused ultrasound signal to the tissue based on the adjusted second output condition through the focused ultrasound transducer.

The adjusting of the output condition to the second output condition may include checking a cavitation detection value in a first frequency interval of a first cycle when outputting the focused ultrasound signal based on the first output condition during the first cycle, and reducing the number of first pulses in a first frequency interval and increasing the number of second pulses in a second frequency interval as a second output condition of a second cycle when the cavitation detection value becomes equal to or exceeds a boiling cavitation limit value, each cycle may include a first frequency interval consisting of at least one first pulse and a second frequency interval consisting of at least one second pulse, and the first frequency interval may be a thermal period for thermal effects, while the second frequency interval may be a boiling period for mechanical effects.

The adjusting of the output condition to the second output condition may include checking a cavitation detection value in a first frequency interval of a first cycle when outputting the focused ultrasound signal based on the first output condition during the first cycle, reducing the total output time of a second cycle as a second output condition of the second cycle when the cavitation detection value becomes equal to or exceeds a boiling cavitation threshold value, increasing the number of first pulses in a first frequency interval and reducing the number of second pulses in a second frequency interval as the second output condition of the second cycle when the cavitation detection value falls between the boiling cavitation threshold value and a non-thermal cavitation limit value, maintaining the number of first pulses in the first frequency interval and the number of second pulses in the second frequency interval as the second output condition of the second cycle when the cavitation detection value falls between the non-thermal cavitation limit value and a non-thermal cavitation threshold value, and reducing the number of first pulses in the first frequency interval and increasing the number of second pulses in the second frequency interval as the second output condition of the second cycle when the cavitation detection value is less than or equal to the non-thermal cavitation threshold value, each cycle may include a first frequency interval consisting of at least one first pulse and a second frequency interval consisting of at least one second pulse, and the first frequency interval may be a thermal period for thermal effects, while the second frequency interval may be a boiling period for mechanical effects.

The adjusting of the output condition to the second output condition may include checking each of a cavitation detection value in a first frequency interval of a first cycle and a cavitation detection value in a second frequency interval of the first cycle when primarily outputting the focused ultrasound signal targeting a first region, and performing control to move to a new second region and output the focused ultrasound signal when the cavitation detection value in the first frequency interval is greater than or equal to a boiling cavitation threshold value and the cavitation detection value in the second frequency interval is greater than or equal to a non-thermal cavitation threshold value, and in other cases, secondarily output the focused ultrasound signal to the first region, each cycle may include a first frequency interval consisting of at least one first pulse and a second frequency interval consisting of at least one second pulse, and the first frequency interval may be a thermal period for thermal effects, while the second frequency interval may be a boiling period for mechanical effects.

The adjusting of the output condition to the second output condition may include checking each of a cavitation detection value in a first frequency interval of a first cycle and a cavitation detection value in a second frequency interval of the first cycle when primarily outputting the focused ultrasound signal targeting a first region, performing control to move to a new second region and output the focused ultrasound signal when the cavitation detection value in the first frequency interval or the cavitation detection value in the second frequency interval is greater than or equal to a non-thermal cavitation threshold value, and in other cases, secondarily output the focused ultrasound signal to the first region, each cycle may include a first frequency interval consisting of at least one first pulse and a second frequency interval consisting of at least one second pulse, and the first frequency interval may be a thermal period for thermal effects, while the second frequency interval may be a boiling period for mechanical effects.

Other features and aspects will be apparent from the following detailed description, the drawings, and the claims.

## BRIEF DESCRIPTION OF THE DRAWINGS

FIG. 1 is a diagram illustrating the configuration of a focused ultrasound processing apparatus according to an embodiment of the present invention;

FIG. 2 is a flowchart illustrating a focused ultrasound processing method according to an embodiment of the present invention;

FIG. 3 is a diagram showing cavitation limit values and cavitation threshold values to be compared with a cavitation detection value according to an embodiment of the present invention;

FIG. 4 is a diagram illustrating signal waveforms for explaining an example of adjusting an output time of a focused ultrasound signal according to an embodiment of the present invention; and

FIG. 5 is a diagram illustrating in more detail signal waveforms for explaining an example of adjusting an output time of a focused ultrasound signal according to an embodiment of the present invention.

Throughout the drawings and the detailed description, unless otherwise described, the same drawing reference numerals will be understood to refer to the same elements, features, and structures. The relative size and depiction of these elements may be exaggerated for clarity, illustration, and convenience.

## DETAILED DESCRIPTION

The advantages and features of the present invention and the manner of achieving the advantages and features will become apparent with reference to embodiments described in detail below together with the accompanying drawings. However, the present invention may be implemented in many different forms and should not be construed as being limited to the embodiments set forth herein, and the embodiments are provided such that this disclosure will be thorough and complete and will fully convey the scope of the present invention to those skilled in the art, and the present invention is defined only by the scope of the appended claims. The same reference numerals refer to the same components throughout this disclosure.

In the following description of the embodiments of the present invention, if a detailed description of related known functions or configurations is determined to unnecessarily obscure the gist of the present invention, the detailed description thereof will be omitted herein. The terms described below are defined in consideration of the functions in the embodiments of the present invention, and these terms may be varied according to the intent or custom of a user or an operator. Therefore, the definitions of the terms used herein should follow contexts disclosed herein.

Hereinafter, embodiments of the present invention will be described in detail with reference to the accompanying drawings. However, the present invention may be realized in various forms, and the scope of the present invention is not limited to such embodiments. The embodiments of the present invention are provided to aid those skilled in the art in the explanation and the understanding of the present invention.

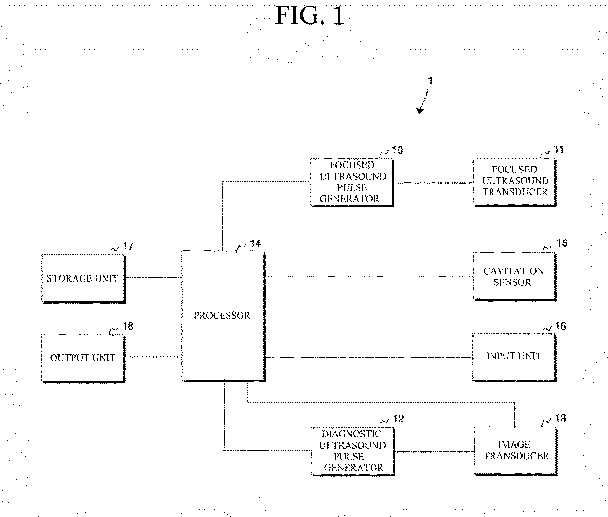

FIG. 1 is a block diagram illustrating the configuration of a focused ultrasound device according to an embodiment of the present invention.

A focused ultrasound processing apparatus 1 according to an embodiment may mechanically ablate soft tissue using not only the thermal effects of a focused ultrasound signal but also the mechanical effects of the focused ultrasound signal. The technique called "focused ultrasound ablation of soft tissue" or "boiling histotripsy" is a method that uses significantly more powerful pressures, dozens of times greater than acoustic pressures used in focused ultrasound signals, at the focal point to artificially induce acoustic cavitation, mechanically fragmenting tissues.

However, according to the focused ultrasound tissue ablation technique, there may occur a shock scattering effect where secondary microbubbles are created simultaneously not only at the focal point but also in its vicinity. While the focused ultrasound tissue ablation technique has the advantage of mechanically breaking down tissues, the precision is compromised due to the shock scattering effect, which makes it challenging to apply when ablating tissues or tumors closely located to vital organs and blood vessels. The focused ultrasound processing apparatus 1 may fragment tissues without inducing a shock scattering effect in the focused ultrasound tissue ablation technique.

Hereinafter, the configuration of a focused ultrasound processing apparatus 1 having the aforementioned characteristics will be described with reference to FIG. 1.

Referring to FIG. 1, the focused ultrasound processing apparatus 1 includes a focused ultrasound pulse generator 10, a focused ultrasound transducer 11, a diagnostic ultrasound pulse generator 12, an image transducer 13, a processor 14, a cavitation sensor 15, an input unit 16, a storage unit 17, and an output unit 18.

The focused ultrasound pulse generator 10 generates a pulse-shaped driving signal having a pulse repetition frequency (PRF) and a duty, and transmits the generated focused ultrasound signal to the focused ultrasound transducer 11.

The focused ultrasound transducer 11 converts an electrical waveform, which is the driving signal received from the focused ultrasound pulse generator 10, into a focused ultrasound signal and outputs the converted focused ultrasound signal to a tissue. At this time, localized tissue temperature elevation occurs due to the delivery of ultrasound energy within the tissue. The focused ultrasound signal may be high-intensity focused ultrasound (HIFU).

The image transducer 13, according to an electrical signal of short pulses, which is a driving signal received from the diagnostic ultrasound pulse generator 12, outputs an image ultrasound signal into the tissue and receives an ultrasound echo signal reflected from the tissue. The image transducer 13 receives the ultrasound echo signal until the next pulse is generated and transmits it to the processor 14.

The focused ultrasound processing apparatus 1 may have a structure where the image transducer 13 is located at the center and the focused ultrasound transducer 11 is arranged in the periphery. However, the structure of the image transducer 13 and the focused ultrasound transducer 11 is not limited to this and may be modified in various ways.

The focused ultrasound processing apparatus 1 according to one embodiment may perform therapeutic operations using the focused ultrasound of the focused ultrasound transducer 11 while obtaining diagnostic images using the imaging ultrasound of the image transducer 13.

A cavitation phenomenon may occur within the tissue due to the focused ultrasound signals output from the focused ultrasound transducer 11. The cavitation phenomenon involves the formation, expansion, and subsequent collapse of small bubbles, driven by the interplay of negative and positive pressures resulting from pressure fluctuations within the tissue as the ultrasound signal interacts with the tissue. The repeated process of bubble expansion and collapse leads to the destruction of cells within the tissue. A cavitation-induced bubble may vibrate and collapse in response to acoustic pressure fluctuations, creating a shock wave. The boiling histotripsy technique may utilize these shock waves caused by the cavitation phenomenon to fragment tissue.

The cavitation sensor 15 detects a cavitation signal, generated by the cavitation phenomenon, from the tissue.

According to the boiling histotripsy, a shock scattering effect may occur in which secondary microbubbles are created simultaneously not only at the focal point of the tissue but also in its vicinity. Bubble clouds caused by the unintended shock scattering effect may lead to shock propagation at points other than the focal location and result in damage to unintended tissue areas.

Therefore, the focused ultrasound processing apparatus 1 according to an embodiment aims to fragment tissue without inducing a shock scattering effect. For example, the processor 14 may analyze a cavitation detection signal detected by the cavitation sensor 15 and adjust at least one of the output time or frequency of the focused ultrasound signal based on the analysis result. By doing so, the shock scattering effect may be prevented. The processor 14 may precisely control bubble dynamics, ensuring no shock scattering effect by adjusting at least one of the output time or frequency of the focused ultrasound signal through the analysis of the cavitation detection signal.

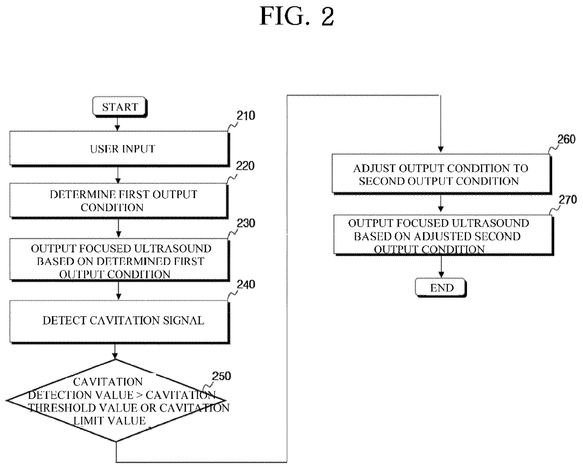

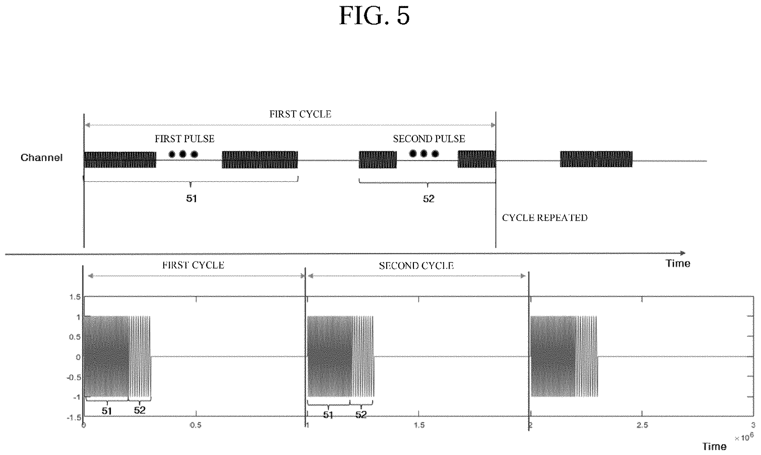

More specifically, the processor 14 may compare a cavitation detection value obtained through the cavitation sensor 15 with a boiling cavitation limit value, and adjust at least one of the output time or frequency of the focused ultrasound signal in order to decrease the cavitation detection value when the cavitation detection value is greater than or equal to the boiling cavitation limit value. An embodiment thereof will be described below with reference to FIGS. 2 and 5.

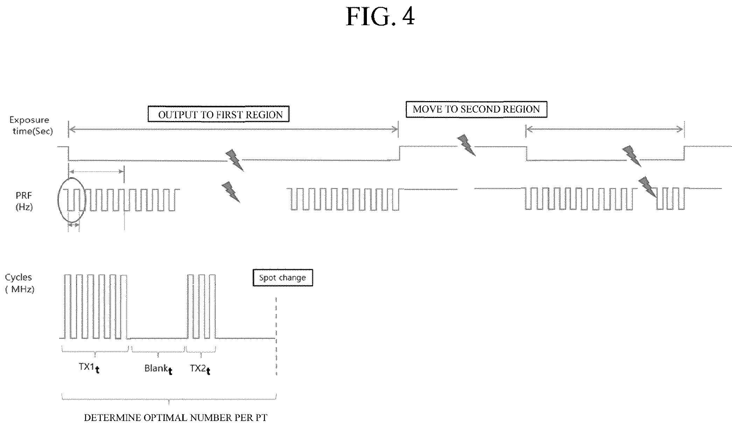

The processor 14 may determine operations, according to the magnitude of the cavitation signal, including primary and secondary output of the focused ultrasound signal to a first region, movement from the first region to the second region (subsequent tissue area) and output of the focused ultrasound signal, stop of output of the focused ultrasound signal, and maintenance of the output time of the focused ultrasound signal. An embodiment thereof will be described below with reference to FIGS. 2 and 4.

The processor 14 may analyze an image ultrasound signal and adjust at least one of the output time or frequency of the focused ultrasound signal based on the analysis result. To this end, the processor 14 receives an ultrasound echo signal from the image transducer 13 and converts the received echo signal into a digital signal, followed by image processing, to create an ultrasound image signal. At this time, the processor 14 may analyze the generated ultrasound image signal and adjust at least one of the output time or frequency of the focused ultrasound signal based on the analysis result. An embodiment thereof will be described below with reference to FIGS. 2 and 5.

The output unit 18 outputs an ultrasound image. The storage unit 17 stores necessary information for signal analysis by the processor 14 or stores information analyzed through the processor 14.

FIG. 2 is a flowchart illustrating a focused ultrasound processing method according to an embodiment of the present invention.

Referring to FIG. 1 and FIG. 2, the focused ultrasound processing apparatus 1 receives tissue characteristics and treatment information from a user in 210. The tissue characteristics information may include tissue thickness information. The treatment information may include treatment depth and treatment range.

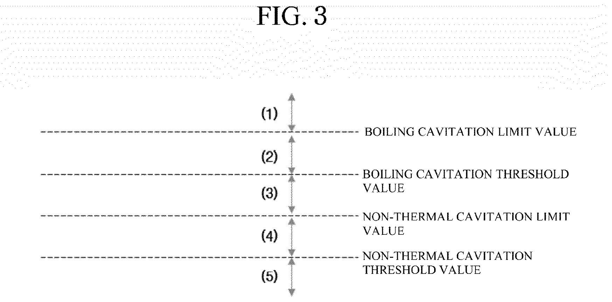

Subsequently, the focused ultrasound processing apparatus 1 determines a first output condition, including at least one of the output time or frequency of the focused ultrasound signal, by reflecting the tissue characteristics and treatment information in 220. Furthermore, the focused ultrasound processing apparatus 1 may set a boiling cavitation threshold value, a boiling cavitation limit value, a non-thermal cavitation threshold value, a non-thermal cavitation limit value, and the like to determine the first output condition. Each of the aforementioned cavitation values will be described below with reference to FIG. 3. The focused ultrasound processing apparatus 1 may store the first output condition in the storage unit 17.

Then, the focused ultrasound processing apparatus 1 outputs the focused ultrasound signal to the tissue based on the determined first output condition through the focused ultrasound transducer 11 in 230.

Subsequently, the focused ultrasound processing apparatus 1 detects a cavitation signal resulting from the cavitation phenomenon occurring within the tissue due to the output focused ultrasound signal, through the cavitation sensor 15 in 240.

Subsequently, the focused ultrasound processing apparatus 1 analyzes a cavitation detection signal detected through the cavitation sensor 15 and adjusts an output condition, which includes at least one of the output time or frequency of the focused ultrasound signal, to a second output condition based on the analysis result.

For example, the focused ultrasound processing apparatus 1 checks whether the cavitation detection value obtained through the cavitation sensor 15 is greater than or equal to the boiling cavitation limit value by comparing the cavitation signal detected through the cavitation sensor 15 with the boiling cavitation limit value in 250. If the cavitation detection value equals or exceeds the boiling cavitation limit value, at least one of the output time or frequency of the focused ultrasound signal is adjusted to the second output condition in 260.

Then, the focused ultrasound processing apparatus 1 outputs the focused ultrasound signal to the tissue based on the adjusted second output condition through the focused ultrasound transducer 11 in 270.

Below, an embodiment of adjusting an output time as an output condition is described.

Firstly, a focused ultrasound output period is composed of multiple cycles, each cycle comprising a first frequency interval consisting of at least one first pulse and a second frequency interval consisting of at least one second pulse. Here, the first frequency interval may be a thermal period for thermal effects, while the second frequency interval may be a boiling period for mechanical effects.

In operation 260, where the output condition is adjusted to the second output condition, the focused ultrasound processing apparatus 1 compares the cavitation detection value, which is detected by the cavitation sensor with a predetermined cavitation threshold value or cavitation limit value. According to the comparison result, the focused ultrasound processing apparatus 1 may adjust at least one of the output time or frequency of the focused ultrasound signal to reduce the cavitation detection value. This method may be categorized into a method using boiling conditions and a method not using boiling conditions.

As an example of a method using boiling conditions, the focused ultrasound processing apparatus 1 compares the cavitation detection value with the boiling cavitation limit value. If the cavitation detection value equals or exceeds the boiling cavitation limit value, the focused ultrasound processing apparatus 1 may adjust at least one of the output time or frequency of the focused ultrasound signal in order to decrease the cavitation detection value.

As an example of a method not using boiling conditions, the focused ultrasound processing apparatus 1 may compare the cavitation detection value with at least one of the boiling cavitation threshold value, the non-thermal cavitation limit value, or the non-thermal cavitation threshold value. Based on the comparison result, the focused ultrasound processing apparatus 1 may adjust at least one of the output time or frequency of the focused ultrasound signal. An embodiment thereof will be described below with reference to FIG. 5.

Below, an embodiment of adjusting a frequency as an output condition is described.

In operation 220, where the first output condition is determined, the focused ultrasound processing apparatus 1 determines a first frequency for maximizing the thermal effect of the focused ultrasound signal before heat generation and a second frequency for maximizing the mechanical effect of the focused ultrasound signal after heat generation.

Subsequently, in operation 230, where the focused ultrasound signal is output based on the first output condition, the focused ultrasound processing apparatus 1 outputs focused ultrasound of the first frequency to the tissue.

Thereafter, in operation 260, where the output condition is adjusted to the second output condition, the focused ultrasound processing apparatus 1 compares the cavitation detection value with the boiling cavitation limit value and the non-thermal cavitation limit value. If the cavitation detection value is greater than or equal to at least one of the boiling cavitation limit value or the non-thermal cavitation limit value, the focused ultrasound processing apparatus 1 adjusts the frequency of the focused ultrasound signal from the first frequency to the second frequency.

Subsequently, in operation 270, where the focused ultrasound signal is output based on the second output condition, the focused ultrasound processing apparatus 1 outputs the adjusted focused ultrasound of the second frequency to the tissue.

Below, an embodiment in which the focused ultrasound processing apparatus 1 determines the first frequency for maximizing the thermal effect of the focused ultrasound signal before heat generation is described.

Tissues exhibit an increasing absorption coefficient $\alpha(f)$ as the frequency increases.

$\alpha(f) = (\alpha_0 f)$, where $\alpha_0$ represents the absorption coefficient at 1 MHz, and $f$ represents a frequency in megahertz (MHz) units.

Depending on the treatment depth, there exists a frequency at which the maximum absorption coefficient is observed.

The heat generation rate Q(z) is as follows:

$$Q(z) = 2(\alpha f)I_0 \exp(-2\alpha f z)$$

Heat generation rate

Here, $\alpha$ represents the absorption coefficient, z represents the treatment depth, and *f* represents the frequency in MHz units.

The differentiation of the heat generation rate Q(z) is as follows:

$$\frac{dQ}{df} = 2\alpha(1 - 2\alpha f z)I_0 \exp(-2\alpha f z) = 0$$

The first frequency, at which the derivative of the heat generation rate Q(z) becomes zero, is a function of the absorption coefficient $\alpha$ and the treatment depth z.

$$f = \frac{1}{1 - 2\alpha z}$$

Hereinafter an example in which the focused ultrasound processing apparatus 1 determines a second frequency to maximize the mechanical effect of the focused ultrasound signal after heat generation is described.

The focused ultrasound processing apparatus 1 may use a mechanical index as follows to determine the second frequency f.

Mechanical $\text{Index} = \dfrac{P_r}{\sqrt{f}}$ , where $P_r$ represents a rarefactional pressure and *f* represents the frequency.

On the other hand, the focused ultrasound processing apparatus 1 not only detects the cavitation signal but also performs additional analysis of an ultrasound image signal. Based on the analysis results of the image signal, the focused ultrasound processing apparatus 1 may adjust at least one of the output time or frequency of the focused ultrasound signal. An embodiment thereof will be described below with reference to FIG. 5.

FIG. 3 is a diagram showing cavitation limit values and cavitation threshold values to be compared with a cavitation detection value according to an embodiment of the present invention.

Referring to FIG. 3, the focused ultrasound processing apparatus 1 may compare the cavitation detection value with each of the boiling cavitation limit value, the boiling cavitation threshold value, the non-thermal cavitation limit value, and the non-thermal cavitation threshold value.

The segments for comparison with the cavitation detection value are divided into the following segments: segment 1 greater than or equal to the boiling cavitation limit value, segment 2 between the boiling cavitation limit value and the boiling cavitation threshold value, segment 3 between the boiling cavitation threshold value and the non-thermal cavitation limit value, segment 4 between the non-thermal cavitation limit value and the non-thermal cavitation threshold value, and segment 5 less than or equal to the non-thermal cavitation threshold value.

The boiling cavitation limit value, the boiling cavitation threshold value, the non-thermal cavitation limit value, and the non-thermal cavitation threshold value may be pre-set by reflecting the input tissue characteristics and treatment information and may be changed by the user.

FIG. 4 is a diagram illustrating signal waveforms for explaining an example of adjusting an output time of a focused ultrasound signal according to an embodiment of the present invention.

Referring to FIGS. 1 and 4, the focused ultrasound processing apparatus 1 may determine operations, according to the magnitude of the cavitation signal, including primary and secondary output of the focused ultrasound signal to a first region, movement from the first region to the second region (subsequent tissue area) and output of the focused ultrasound signal, stop of output of the focused ultrasound signal, and maintenance of the output time of the focused ultrasound signal.

Firstly, a focused ultrasound output period is composed of multiple cycles, each cycle comprising a first frequency interval consisting of at least one first pulse and a second frequency interval consisting of at least one second pulse. Here, the first frequency interval is a thermal period for thermal effects, while the second frequency interval is a boiling period for mechanical effects.

In an example of using boiling conditions, the focused ultrasound processing apparatus 1 moves to a new second region and outputs the focused ultrasound signal if the cavitation detection value in the first frequency interval targeting the first region is greater than or equal to the boiling cavitation threshold value and the cavitation detection value in the second frequency interval is greater than or equal to the non-thermal cavitation threshold value.

In other cases, the focused ultrasound processing apparatus 1 primarily outputs the focused ultrasound signal to the first region, and then secondarily outputs the focused ultrasound signal to the first region. This embodiment illustrates an example where boiling conditions are utilized for tissue ablation.

In an example of using no boiling conditions, the focused ultrasound processing apparatus 1 moves to a new second region and outputs the focused ultrasound signal if the cavitation detection value in the first frequency interval targeting the first region or in the second frequency interval is greater than or equal to the non-thermal cavitation threshold value. In other cases, the focused ultrasound processing apparatus 1 may secondarily output the focused ultrasound signal to the first region. This example illustrates an example where boiling conditions are not utilized for tissue ablation.

Meanwhile, the focused ultrasound processing apparatus 1, when performing secondary output of the focused ultrasound signal to the first region during the second cycle after primary output to the first region during the first cycle, may adjust the frequency or output time of the second cycle.

For example, the focused ultrasound processing apparatus 1 may generate a focused ultrasound signal composed of a combination of N first pulses for thermal effects and M second pulses for mechanical effects during the first cycle, and output the generated focused ultrasound signal. During the second cycle, the focused ultrasound processing apparatus 1 may vary the combination of N and M to adjust the focused ultrasound signal while maintaining the total number of pulses, and output the adjusted focused ultrasound signal. Further details on this will be described below with reference to FIG. 5.

FIG. 5 is a diagram illustrating in more detail signal waveforms for explaining an example of adjusting an output time of a focused ultrasound signal according to an embodiment of the present invention.

A focused ultrasound output period is composed of multiple cycles, each cycle comprising a first frequency interval consisting of at least one first pulse 51 and a second frequency interval consisting of at least one second pulse 52. Here, the first frequency interval is a thermal period for thermal effects, while the second frequency interval is a boiling period for mechanical effects.

Referring to FIGS. 1 and 5, the focused ultrasound processing apparatus 1 outputs a focused ultrasound signal by combining the first pulse for thermal effects of the ultrasound signal and the second pulse for mechanical effects.

For example, the focused ultrasound processing apparatus 1 may generate N (N is a positive integer) first pulses 51 and M (M is a positive integer) second pulses 52 per cycle, forming N first pulses 51 and M second pulses 52 into a single pulse, which is then output one per cycle through the focused ultrasound transducer.

The first pulse 51 may be a long burst pulse signal, and the second pulse 52 may be a short burst pulse signal. The first pulse 51 may include fh frequency and pulse length Ph frequency for thermal effects. The second pulse 52 may include fm frequency and pulse length Pm frequency for mechanical effects.

In this case, the focused ultrasound processing apparatus 1 may combine various frequencies, including combinations where fh ≠ fm and Ph t- Pm, fh = fm and Ph ≠ Pm, or fh ≠ fm and Ph = Pm.

The focused ultrasound processing apparatus 1 checks a cavitation detection value within the first frequency interval of the first cycle. Subsequently, if the cavitation detection value is greater than or equal to the boiling cavitation limit value (segment 1 shown in FIG. 3), the focused ultrasound processing apparatus 1 decreases the number of first pulses 51 within the first frequency interval and increasing the number of second pulses 52 within the second frequency interval in the second cycle to generate and output the focused ultrasound signal.

For example, if the cavitation detection value is greater than or equal to the boiling cavitation limit value at the point where N-a first pulses 51 among the N first pulses are output in the first cycle, the focused ultrasound processing apparatus 1 generates N-a first pulses 51 by reducing the number of first pulses 51 and generates the remaining M+a second pulses 52 and outputs the generated pulses. This approach illustrates an example of utilizing boiling conditions.

As another example, an embodiment in which boiling conditions are not utilized will be described.

If the cavitation detection value is greater than or equal to the boiling cavitation threshold value (segment 2 shown in FIG. 3), the focused ultrasound processing apparatus 1 outputs the focused ultrasound signal by reducing the total output time of the second cycle.

In contrast, when the cavitation detection value falls between the boiling cavitation threshold value and the non-thermal cavitation limit value (segment 3 shown in FIG. 3), the focused ultrasound processing apparatus 1 outputs the focused ultrasound signal by increasing the number of first pulses 51 in the first frequency interval of the second cycle and reducing the number of second pulses 52 in the second frequency interval.

If the cavitation detection value falls between the non-thermal cavitation limit value and the non-thermal cavitation threshold value (segment 4 shown in FIG. 3), the focused ultrasound processing apparatus 1 outputs the focused ultrasound signal while maintaining the number of first pulses 51 in the first frequency interval and the number of second pulses 52 in the second frequency interval of the second cycle.

If the cavitation detection value is less than or equal to the non-thermal cavitation threshold value (segment 5 shown in FIG. 3), the focused ultrasound processing apparatus 1 outputs the focused ultrasound signal by reducing the number of first pulses 51 in the first frequency interval of the second cycle and increasing the number of second pulses 52 in the second frequency interval.

Accordingly, the focused ultrasound processing apparatus 1 may generate bubbles locally and control the size and duration of the bubbles to enhance the accuracy of the treatment.

Additionally, the focused ultrasound processing apparatus 1 may further analyze an ultrasound image signal and adjust the output time and frequency of the focused ultrasound signal based on the analysis results.

As an example of using boiling conditions, the focused ultrasound processing apparatus 1 checks an image brightness value of the focal area within the first frequency interval of the first cycle through the analysis of an image signal during the first cycle. In this case, if the image brightness value of the focal area becomes equal to or exceeds a boiling cavitation image brightness threshold value, the focused ultrasound processing apparatus 1 outputs the focused ultrasound signal by reducing the number of first pulses 51 in the first frequency interval of the second cycle and increasing the number of second pulses 52 in the second frequency interval.

As an example of not using boiling conditions, the focused ultrasound processing apparatus 1 checks an image brightness value of the focal area within the first frequency interval of the first cycle through the analysis of an image signal during the first cycle. In this case, if the image brightness value of the focal area becomes equal to or exceeds a boiling cavitation image brightness value, the focused ultrasound processing apparatus 1 outputs the focused ultrasound signal by reducing the total output time of the second cycle.

In contrast, when the image brightness value of the focal area falls between the boiling cavitation image brightness value and a non-thermal cavitation image limit value, the focused ultrasound processing apparatus 1 outputs the focused ultrasound signal by increasing the number of first pulses 51 in the first frequency interval of the second cycle and reducing the number of second pulses 52 in the second frequency interval.

In contrast, when the image brightness value of the focal area falls between the non-thermal cavitation image brightness value and a non-thermal cavitation image threshold value, the focused ultrasound processing apparatus 1 outputs the focused ultrasound signal while maintaining the number of first pulses 51 in the first frequency interval of the second cycle and the number of second pulses 52 in the second frequency interval.

In this case, if the image brightness value of the focal area is less than or equal to the non-thermal cavitation image brightness threshold value, the focused ultrasound processing apparatus 1 outputs the focused ultrasound signal by reducing the number of first pulses 51 in the first frequency interval of the second cycle and increasing the number of second pulses 52 in the second frequency interval.

According to an exemplary embodiment of the focused ultrasound processing apparatus and method thereof, tissue ablation with focused ultrasound may be achieved more safely and effectively beyond the limits of thermal effects by applying mechanical effects.

Furthermore, when applying mechanical effects to focused ultrasound tissue ablation, the pressure and intensity of the focused ultrasound signal may be reduced by adjusting the frequency or output time of the focused ultrasound signal using cavitation signals and ultrasound image signals, thereby enabling tissue ablation without inducing a shock scattering effect. Moreover, it is possible to enhance the accuracy of the treatment by locally generating bubbles and controlling the size and duration of the bubbles.

Heretofore, the present invention has been described by focusing on the exemplary embodiments. It can be understood by those skilled in the art to which the present invention pertains that the present invention can be implemented in modified forms without departing from the essential feature of the present invention. Therefore, the disclosed embodiments should be considered as illustrative rather than determinative. The scope of the present invention is defined by the appended claims rather than by the foregoing description, and all differences within the scope of equivalents thereof should be construed as being included in the present invention.

**Claims**

1. A focused ultrasound processing apparatus comprising:

   a focused ultrasound transducer configured to output a focused ultrasound signal to tissue;
   a cavitation sensor configured to detect a cavitation signal resulting from a cavitation phenomenon occurring in the tissue due to the focused ultrasound signal; and
   a processor configured to analyze the cavitation signal detected by the cavitation sensor and adjust at least one of output time or frequency of the focused ultrasound signal based on an analysis result.

2. The focused ultrasound processing apparatus of claim 1, wherein the processor is configured to compare a cavitation detection value obtained through the cavitation sensor with a predetermined cavitation threshold value or a cavitation limit value, and based on the comparison result, adjust at least one of the output time or frequency of the focused ultrasound signal in order to decrease the cavitation value.

3. The focused ultrasound processing apparatus of claim 2, wherein the processor is configured to compare the cavitation detection value with a boiling cavitation limit value and adjust at least one of the output time or frequency of the focused ultrasound signal in order to decrease the cavitation detection value when the cavitation detection value is greater than or equal to the boiling cavitation limit value.

4. The focused ultrasound processing apparatus of claim 2, wherein the processor is configured to compare the cavitation detection value obtained through the cavitation sensor with at least one of a boiling cavitation threshold value, a non-thermal cavitation limit value, or a non-thermal cavitation threshold value and adjust at least one of the output time or frequency of the focused ultrasound signal based on the comparison result.

5. The focused ultrasound processing apparatus of claim 1, wherein the processor is configured to control output of the focused ultrasound signal by repeating multiple cycles, each cycle comprises a first frequency interval consisting of at least one first pulse and a second frequency interval consisting of at least one second pulse, and the first frequency interval is a thermal period for thermal effects, while the second frequency interval is a boiling period for mechanical effects.

6. The focused ultrasound processing apparatus of claim 5, wherein the first frequency interval comprises fh frequency and Ph frequency for thermal effects and the second frequency interval comprises fm frequency and Pm frequency for mechanical effects.

7. The focused ultrasound processing apparatus of claim 5, wherein the processor is configured to check a cavitation detection value within a first frequency interval of a first cycle and perform control to reduce the number of first pulses in the first frequency interval of a second cycle following the first cycle and increase the number of second pulses of a second frequency interval of the second cycle when the cavitation detection value becomes equal to or exceeds the boiling cavitation limit value.

8. The focused ultrasound processing apparatus of claim 5, wherein the processor is configured to check a cavitation detection value within a first frequency interval of a first cycle and perform control

to reduce the total output time of a second cycle when the cavitation detection value becomes equal to or exceeds a boiling cavitation threshold value,
to increase the number of first pulses in a first frequency interval of the second cycle and reduce the number of second pulses in a second frequency interval of the second cycle when the cavitation detection value falls between the boiling cavitation threshold value and a non-thermal cavitation limit value,
to maintain the number of first pulses in the first frequency interval of the second cycle and the number of second pulses in the second frequency interval of the second cycle when the cavitation detection value falls between the non-thermal cavitation limit value and a non-thermal cavitation threshold value, and
to reduce the number of first pulses in the first frequency interval of the second cycle and increase the number of second pulses in the second frequency interval of the second cycle when the cavitation detection value is less than or equal to the non-thermal cavitation threshold value.

9. The focused ultrasound processing apparatus of claim 5, wherein the processor is configured to check each of a cavitation detection value in a first frequency interval of a first cycle and a cavitation detection value in a second frequency interval of the first cycle when primarily outputting the focused ultrasound signal to a first region, and perform control to move to a new second region output the focused ultrasound signal when the cavitation detection value in the first frequency interval is greater than or equal to a boiling cavitation threshold value and the cavitation detection value in the second frequency interval is greater than or equal to a non-thermal cavitation threshold value, and
in other cases, the processor is configured to perform control to secondarily output the focused ultrasound signal to the first region.

10. The focused ultrasound processing apparatus of claim 5, wherein the processor is configured to check each of a cavitation detection value in a first frequency interval of a first cycle and a cavitation detection value in a second frequency interval of the first cycle when primarily outputting the focused ultrasound signal to a first region, and may perform control to move to a new second region and output the focused ultrasound signal when the cavitation detection value in the first frequency interval or the cavitation detection value in the second frequency interval is greater than or equal to a non-thermal cavitation threshold value, and

in other cases, the processor is configured to perform control to secondarily output the focused ultrasound signal to the first region.

11. The focused ultrasound processing apparatus of claim 1, further comprising:

an input unit configured to receive tissue characteristics and treatment information,
wherein the processor is configured to determine a cavitation limit value, a cavitation threshold value, and the output time and frequency of the focused ultrasound signal by reflecting the input tissue characteristics and treatment information.

12. The focused ultrasound processing apparatus of claim 1, wherein the processor is configured to determine a first frequency for maximizing thermal effect of the focused ultrasound signal before heat generation and a second frequency for maximizing mechanical effect of the focused ultrasound signal after heat generation.

13. The focused ultrasound processing apparatus of claim 12, wherein the processor is configured to control a focused ultrasound signal having the determined first frequency to be output through the focused ultrasound transducer, compare a cavitation detection value with at least one of a boiling cavitation limit value or a non-thermal cavitation limit value, and when the cavitation detection value is greater than or equal to the non-thermal cavitation limit value or the boiling cavitation limit value, perform control to adjust a frequency of the focused ultrasound signal from the first frequency to the second frequency and then output the adjusted focused ultrasound signal having the second frequency through the focused ultrasound transducer.

14. The focused ultrasound processing apparatus of claim 1, further comprising:

an image transducer configured to output an image ultrasound signal to a tissue and receive an ultrasound echo signal reflected from the tissue,
wherein the processor is configured to generate an ultrasound image signal by signalprocessing the received ultrasound echo signal, analyze the generated ultrasound image signal, and adjust at least one of the output time or frequency of the focused ultrasound signal based on an analysis result.

15. The focused ultrasound processing apparatus of claim 14, wherein the processor is configured to control output of the focused ultrasound signal by repeating multiple cycles,

each cycle comprises a first frequency interval consisting of at least one first pulse and a second frequency interval consisting of at least one second pulse,
the first frequency interval is a thermal period for thermal effects, while the second frequency interval is a boiling period for mechanical effects, and
the processor is configured to check an image brightness value of a focal area in a first frequency interval of a first cycle through analysis of the image signal and perform control to reduce the number of first pulses in a first frequency interval of a second cycle and increase the number of second pulses of a second frequency interval of the second cycle when the image brightness value of the focal area becomes equal to or exceeds a boiling cavitation image brightness limit value.

16. The focused ultrasound processing apparatus of claim 14, wherein the processor is configured to control output of the focused ultrasound signal by repeating multiple cycles,

each cycle comprises a first frequency interval consisting of at least one first pulse and a second frequency interval consisting of at least one second pulse,
the first frequency interval is a thermal period for thermal effects, while the second frequency interval is a boiling period for mechanical effects, and
the processor is configured to check an image brightness value of a focal area in a first frequency interval of a first cycle through analysis of the image signal and
perform control to reduce the total output time of a second cycle when the image brightness value of the focal area becomes equal to or exceeds a boiling cavitation image brightness value,
to increase the number of first pulses in a first frequency interval of the second cycle and reduce the number of second pulses in a second frequency interval of the second cycle when the image brightness value of the focal area falls between the boiling cavitation threshold value and a non-thermal cavitation image brightness limit value,

to maintain the number of first pulses in the first frequency interval of the second cycle and the number of second pulses in the second frequency interval of the second cycle when the image brightness value of the focal area falls between the non-thermal cavitation image brightness limit value and a non-thermal cavitation image brightness threshold value, and

to reduce the number of first pulses in the first frequency interval of the second cycle and increase the number of second pulses in the second frequency interval of the second cycle when the image brightness value of the focal area is less than or equal to the non-thermal cavitation image brightness threshold value.

17. A focused ultrasound processing method using a focused ultrasound processing apparatus, the method comprising:

determining a first output condition including at least one of output time or frequency of a focused ultrasound signal;

outputting a focused ultrasound signal, based on the determined first output condition, through a focused ultrasound transducer;

detecting, by a cavitation sensor, a cavitation signal resulting from a cavitation phenomenon occurring in a tissue due to the output focused ultrasound signal;

analyzing a cavitation detection value obtained through the cavitation sensor and adjusting an output condition including at least one of output time or frequency of a focused ultrasound signal to a second output condition based on an analysis result; and

outputting the focused ultrasound signal to the tissue based on the adjusted second output condition through the focused ultrasound transducer.

18. The focused ultrasound processing method of claim 17, wherein the adjusting of the output condition to the second output condition comprises:

checking a cavitation detection value in a first frequency interval of a first cycle when outputting the focused ultrasound signal based on the first output condition during the first cycle; and

reducing the number of first pulses in a first frequency interval and increasing the number of second pulses in a second frequency interval as a second output condition of a second cycle when the cavitation detection value becomes equal to or exceeds a boiling cavitation limit value,

each cycle comprises a first frequency interval consisting of at least one first pulse and a second frequency interval consisting of at least one second pulse, and

the first frequency interval is a thermal period for thermal effects, while the second frequency interval may be a boiling period for mechanical effects.

19. The focused ultrasound processing method of claim 17, wherein the adjusting of the output condition to the second output condition comprises:

checking a cavitation detection value in a first frequency interval of a first cycle when outputting the focused ultrasound signal based on the first output condition during the first cycle;

reducing the total output time of a second cycle as a second output condition of the second cycle when the cavitation detection value becomes equal to or exceeds a boiling cavitation threshold value;

increasing the number of first pulses in a first frequency interval and reducing the number of second pulses in a second frequency interval as the second output condition of the second cycle when the cavitation detection value falls between the boiling cavitation threshold value and a non-thermal cavitation limit value;

maintaining the number of first pulses in the first frequency interval and the number of second pulses in the second frequency interval as the second output condition of the second cycle when the cavitation detection value falls between the non-thermal cavitation limit value and a non-thermal cavitation threshold value; and

reducing the number of first pulses in the first frequency interval and increasing the number of second pulses in the second frequency interval as the second output condition of the second cycle when the cavitation detection value is less than or equal to the non-thermal cavitation threshold value,

each cycle comprises a first frequency interval consisting of at least one first pulse and a second frequency interval consisting of at least one second pulse, and

the first frequency interval is a thermal period for thermal effects, while the second frequency interval is a boiling period for mechanical effects.

20. The focused ultrasound processing method of claim 17, wherein the adjusting of the output condition to the second output condition comprises:

checking each of a cavitation detection value in a first frequency interval of a first cycle and a cavitation detection value in a second frequency interval of the first cycle when primarily outputting the focused ultrasound signal targeting a first region; and

performing control to move to a new second region and output the focused ultrasound signal when the cavitation detection value in the first frequency interval is greater than or equal to a boiling cavitation threshold value and the cavitation detection value in the second frequency interval is greater than or equal to a non-thermal cavitation threshold value, and in other cases, secondarily output the focused ultrasound signal to the first region,

each cycle comprises a first frequency interval consisting of at least one first pulse and a second frequency interval consisting of at least one second pulse, and

the first frequency interval is a thermal period for thermal effects, while the second frequency interval is a boiling period for mechanical effects.

21. The focused ultrasound processing method of claim 17, wherein the adjusting of the output condition to the second output condition comprises:

checking each of a cavitation detection value in a first frequency interval of a first cycle and a cavitation detection value in a second frequency interval of the first cycle when primarily outputting the focused ultrasound signal targeting a first region; and

performing control to move to a new second region and output the focused ultrasound signal when the cavitation detection value in the first frequency interval or the cavitation detection value in the second frequency interval is greater than or equal to a non-thermal cavitation threshold value, and in other cases, secondarily output the focused ultrasound signal to the first region,

each cycle comprises a first frequency interval consisting of at least one first pulse and a second frequency interval consisting of at least one second pulse, and

the first frequency interval is a thermal period for thermal effects, while the second frequency interval is a boiling period for mechanical effects.

# FIG. 1

1

```
                                  ┌──────────────┐ 10        ┌──────────────┐ 11
                                  │   FOCUSED    │           │   FOCUSED    │
                                  │  ULTRASOUND  │           │  ULTRASOUND  │
                                  │    PULSE     │           │  TRANSDUCER  │
                                  │  GENERATOR   │           │              │
                                  └──────────────┘           └──────────────┘

   ┌──────────────┐ 17   ┌────────────┐ 14                   ┌──────────────┐ 15
   │ STORAGE UNIT │      │            │                      │  CAVITATION  │
   └──────────────┘      │            │                      │    SENSOR    │
                         │ PROCESSOR  │                      └──────────────┘
   ┌──────────────┐ 18   │            │                      ┌──────────────┐ 16
   │ OUTPUT UNIT  │      │            │                      │  INPUT UNIT  │
   └──────────────┘      └────────────┘                      └──────────────┘

                                  ┌──────────────┐ 12        ┌──────────────┐ 13
                                  │  DIAGNOSTIC  │           │    IMAGE     │
                                  │  ULTRASOUND  │           │  TRANSDUCER  │
                                  │    PULSE     │           │              │
                                  │  GENERATOR   │           └──────────────┘
                                  └──────────────┘
```

15

# FIG. 2

```
                    ┌─────────┐
                    │  START  │
                    └────┬────┘
                         │
         ┌───────────────▼───────────────┐  ～210
         │          USER INPUT           │
         └───────────────┬───────────────┘  ～220
                         │
         ┌───────────────▼───────────────┐
         │     DETERMINE FIRST OUTPUT    │
         │           CONDITION           │
         └───────────────┬───────────────┘  ～230
                         │
         ┌───────────────▼───────────────┐
         │    OUTPUT FOCUSED ULTRASOUND  │
         │    BASED ON DETERMINED FIRST  │
         │        OUTPUT CONDITION       │
         └───────────────┬───────────────┘  ～240
                         │
         ┌───────────────▼───────────────┐
         │     DETECT CAVITATION SIGNAL  │
         └───────────────┬───────────────┘
                         │
                    ╱────▼────╲  ～250
              ╱   CAVITATION    ╲
         ╱ DETECTION VALUE > CAVITATION ╲
         ╲ THRESHOLD VALUE OR CAVITATION ╱
              ╲   LIMIT VALUE   ╱
                    ╲─────────╱
```

ADJUST OUTPUT CONDITION TO
SECOND OUTPUT CONDITION  ～260

OUTPUT FOCUSED ULTRASOUND
BASED ON ADJUSTED SECOND
OUTPUT CONDITION  ～270

END

EP 4 458 410 A1

16

# FIG. 3

(1)

--------------------------------------------------- BOILING CAVITATION LIMIT VALUE

(2)

--------------------------------------------------- BOILING CAVITATION THRESHOLD
VALUE

(3)

--------------------------------------------------- NON-THERMAL CAVITATION LIMIT
VALUE

(4)

--------------------------------------------------- NON-THERMAL CAVITATION
THRESHOLD VALUE

(5)

# FIG. 4

| OUTPUT TO FIRST REGION | MOVE TO SECOND REGION |

Exposure time(Sec)

PRF (Hz)

Cycles ( MHz)

Spot change

$TX1_t$    $Blank_t$    $TX2_t$

DETERMINE OPTIMAL NUMBER PER PT

# FIG. 5

FIRST CYCLE

FIRST PULSE

SECOND PULSE

Channel

51

52

CYCLE REPEATED

Time

FIRST CYCLE

SECOND CYCLE

51 52

51 52

Time

19

Europäisches
Patentamt

European
Patent Office

Office européen
des brevets

# EUROPEAN SEARCH REPORT

Application Number

EP 23 19 1565

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/251528 A1 (CANNEY MICHAEL S [US] ET AL) 13 October 2011 (2011-10-13) | 1-4 | INV. A61N7/00 A61N7/02 |
| Y | * paragraphs [0017], [0020] - [0022], | 14 | |
| A | [0029] - [0032], [0037]; claims 20,21,25; figures 1A-2 * | 5-10,12, 13,15-21 | |
| X | US 2019/350557 A1 (SHI WILLIAM TAO [US] ET AL) 21 November 2019 (2019-11-21) * paragraphs [0029], [0030], [0034], [0035], [0042] - [0050]; figures 1-8 * | 1-4 | |
| X | US 2021/169515 A1 (PAHK KI JOO [KR] ET AL) 10 June 2021 (2021-06-10) * paragraphs [0015] - [0018], [0032], [0040], [0048], [0050], [0054], [0073] - [0077]; figures 1-10 * | 1-4 | |
| X | US 2023/139561 A1 (BAR-ZION AVINOAM D [US] ET AL) 4 May 2023 (2023-05-04) * paragraphs [0059], [0103] - [0111], [0141]; figures 1-20 * | 1 | |
| X | US 2020/164231 A1 (CANNATA JONATHAN M [US] ET AL) 28 May 2020 (2020-05-28) * paragraphs [0043], [0143], [0147], [0212] - [0219]; figures 1-17E * | 1 | TECHNICAL FIELDS SEARCHED (IPC) A61N |
| Y | US 2007/038099 A1 (SUGITA NAMI [JP] ET AL) 15 February 2007 (2007-02-15) * paragraphs [0086] - [0108]; figures 1-11 * | 14 | |
| A | US 2016/339273 A1 (AL MAYIAH FARES [SA]) 24 November 2016 (2016-11-24) * paragraphs [0009], [0038] - [0045]; figures 1-7 * | 1-21 | |

The present search report has been drawn up for all claims

| Place of search | Date of completion of the search | Examiner |
|---|---|---|
| The Hague | 25 January 2024 | Viidebaum, Mikk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 1565

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2024

| Patent document cited in search report | | Publication date | Patent family member(s) | | Publication date |
|---|---|---|---|---|---|
| US 2011251528 | A1 | 13-10-2011 | US | 2011251528 A1 | 13-10-2011 |
| | | | US | 2015119763 A1 | 30-04-2015 |
| US 2019350557 | A1 | 21-11-2019 | EP | 3554382 A1 | 23-10-2019 |
| | | | US | 2019350557 A1 | 21-11-2019 |
| | | | US | 2022133277 A1 | 05-05-2022 |
| | | | WO | 2018109652 A1 | 21-06-2018 |
| US 2021169515 | A1 | 10-06-2021 | KR | 20210071603 A | 16-06-2021 |
| | | | US | 2021169515 A1 | 10-06-2021 |
| US 2023139561 | A1 | 04-05-2023 | US | 2020306564 A1 | 01-10-2020 |
| | | | US | 2023139561 A1 | 04-05-2023 |
| | | | WO | 2020198728 A1 | 01-10-2020 |
| US 2020164231 | A1 | 28-05-2020 | AU | 2019389001 A1 | 10-06-2021 |
| | | | CA | 3120586 A1 | 04-06-2020 |
| | | | CN | 113286552 A | 20-08-2021 |
| | | | EP | 3886737 A1 | 06-10-2021 |
| | | | JP | 2022510654 A | 27-01-2022 |
| | | | US | 2020164231 A1 | 28-05-2020 |
| | | | US | 2020346046 A1 | 05-11-2020 |
| | | | US | 2023310900 A1 | 05-10-2023 |
| | | | US | 2023310901 A1 | 05-10-2023 |
| | | | US | 2024001157 A1 | 04-01-2024 |
| | | | US | 2024001158 A1 | 04-01-2024 |
| | | | WO | 2020113083 A1 | 04-06-2020 |
| US 2007038099 | A1 | 15-02-2007 | CN | 1891167 A | 10-01-2007 |
| | | | JP | 4369907 B2 | 25-11-2009 |
| | | | JP | 2007007279 A | 18-01-2007 |
| | | | US | 2007038099 A1 | 15-02-2007 |
| US 2016339273 | A1 | 24-11-2016 | EP | 3099379 A1 | 07-12-2016 |
| | | | GB | 2515134 A | 17-12-2014 |
| | | | US | 2016339273 A1 | 24-11-2016 |
| | | | WO | 2015110836 A1 | 30-07-2015 |

EPO FORM P0459

For more details about this annex : see Official Journal of the European Patent Office, No. 12/82